# EUROPEAN PATENT SPECIFICATION

(11) **EP 4 260 805 B1**
(45) Date of publication and mention of the grant of the patent: **28.01.2026**
(21) Application number: 20965844.2
(22) Date of filing: 14.12.2020
(51) Int. Cl.: A61B 5/318, A61B 5/341, A61B 5/282, A61B 5/00

(54) **MULTI-AXIAL METHOD FOR ACQUIRING CARDIAC SIGNALS AND CORRESPONDING ATTACHMENT TOOL**
MULTI-AXIS-VERFAHREN ZUR ERFASSUNG VON HERZSIGNALEN UND ENTSPRECHENDES ANBAUGERÄT
MÉTHODE MULTI-AXIALE POUR L'ACQUISITION DE SIGNAUX CARDIAQUES ET OUTIL DE FIXATION CORRESPONDANT

(43) Date of publication of application: 18.10.2023
(73) Proprietor: NTT, Inc., Tokyo 100-8116 (JP)
(72) Inventor: TSUKADA, Shingo, Musashino-shi, Tokyo 180-8585 (JP)
(74) Representative: Santarelli
(86) International application number: PCT/JP2020/046536
(87) International publication number: WO 2022/130456

(56) References cited:
- JP-A- 2003 529 407
- JP-A- S61 162 933
- E. FRANK: "An Accurate, Clinically Practical System For Spatial Vectorcardiography", CIRCULATION, vol. 13, no. 5, 1 May 1956 (1956-05-01), pages 737 - 749, XP055033733, ISSN: 0009-7322, DOI: 10.1161/01.CIR.13.5.737
- MALMIVUO JAAKKO ET AL: "Bioelectromagnetism - Principles and Applications of Bioelectric and Biomagnetis Fields", 1 January 1995 (1995-01-01), pages 290 - 306, XP093177566, Retrieved from the Internet <URL:https://www.bem.fi/book/16/16.htm>
- OWENS C ET AL: "Comparison of Value of Leads from Body Surface Maps to 12-Lead Electrocardiogram for Diagnosis of Acute Myocardial Infarction", AMERICAN JOURNAL OF CARDIOLOGY, CAHNERS PUBLISHING CO., NEWTON, MA, US, vol. 102, no. 3, 1 August 2008 (2008-08-01), pages 257 - 265, XP024529077, ISSN: 0002-9149, [retrieved on 20080524], DOI: 10.1016/J.AMJCARD.2008.03.046
- SANO, TOYOMI: "Vectorcardiogram", JAPANESE JOURNAL OF MEDICAL ELECTRONICS AND BIOLOGICAL ENGINEERING, vol. 3, no. 1, 1965, JP , pages 15 - 28, XP009544719, ISSN: 0021-3292

## Description

### [Technical Field]

The present invention relates to a method for acquiring cardiac signals over multi-axis electrode arrangements and a corresponding attachment tool for acquiring cardiac signals.

### [Background Art]

An electrocardiogram is useful information for grasping a condition of a heart, and for example, a use of the electrocardiogram can determine whether or not the subject is in a condition having a high possibility of suffering from a heart disease (NPL 1).

### [Citation List]

### [Non Patent Literature]

[NPL 1] "Recommendations for the standardization and interpretation of the electrocardiogram: part I: The electrocardiogram and its technology: a scientific statement from the American Heart Association Electrocardiography and Arrhythmias Committee, Council on Clinical Cardiology; the American College of Cardiology Foundation; and the Heart Rhythm Society", endorsed by the International Society for Computerized Electrocardiology, Circulation 2007 Mar 13;115(10):1306-1324

### [Summary of Invention]

### [Technical Problem]

However, the information obtained from a waveform of the electrocardiogram may not necessarily be sufficient to grasp the condition of the heart. For example, even if the waveform of the electrocardiogram is similar, the way of onset of the disease related to the heart may be different. As described above, although the information obtained from the waveform of the electrocardiogram may not necessarily be sufficient to grasp the condition of the heart, it may be difficult to obtain other information depending on the disease. For example, in a case of heart failure, if the load on the heart and the cardiac function can be observed by the waveform of the electrocardiogram, the symptoms can be reduced or the onset can be suppressed by early intervention in daily life. In order to further improve the accuracy of suppressing the onset, other information may be acquired by using other techniques such as collecting blood and performing an image examination, but it is not realistic to perform the acquisition of information in daily life. Therefore, depending on the disease, there is a case in which the condition of the heart must be grasped substantially based on only the waveform of the electrocardiogram.

In view of the above circumstances, it is an object of the present invention to provide a technique for increasing the information obtained from the waveform of the electrocardiogram.

### [Solution to Problem]

One aspect of the present invention relates to a method for acquiring cardiac signals according to independent claim 1. Another aspect of the present invention relates to an attachment tool according to independent claim 6.

### [Advantageous Effects of Invention]

According to the present invention, it is possible to provide a technique for increasing information obtained from a waveform of an electrocardiogram.

### [Brief Description of Drawings]

[Fig. 1]

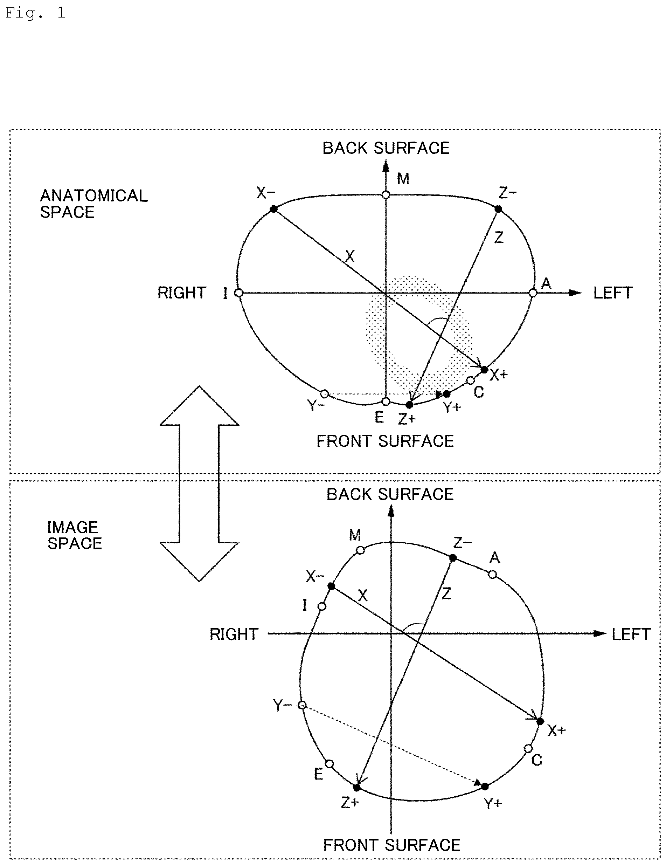

   Fig. 1 is a first explanatory diagram explaining a relationship between information obtained from the waveform of the electrocardiogram and the arrangement of electrodes when acquiring the waveform of the electrocardiogram.
[Fig. 2]

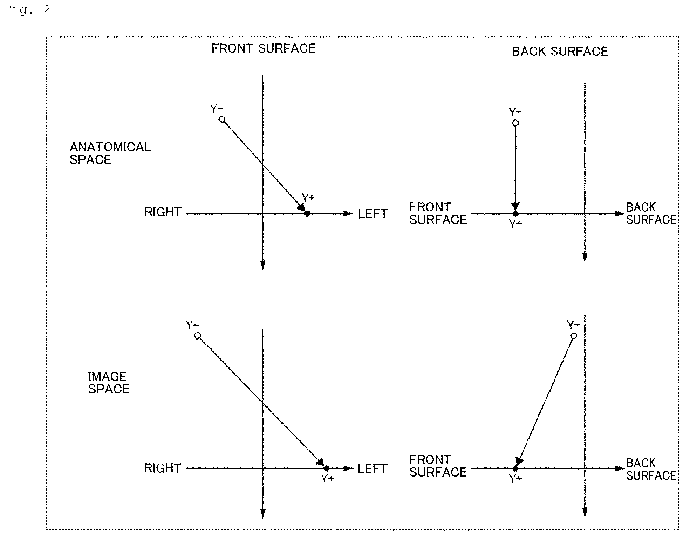

   Fig. 2 is a first explanatory diagram explaining a relationship between information obtained from the waveform of the electrocardiogram and the arrangement of electrodes when acquiring the waveform of the electrocardiogram.
[Fig. 3]

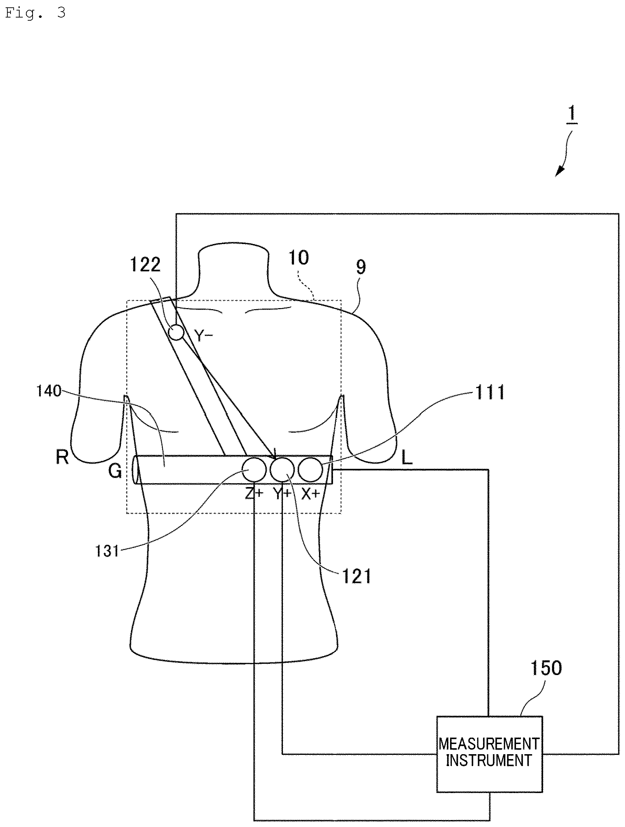

   Fig. 3 is a diagram showing an example of a view of an upper half of a body of a wearer 9 wearing a lead instrument 1 of an embodiment when viewed from a front.
[Fig. 4]

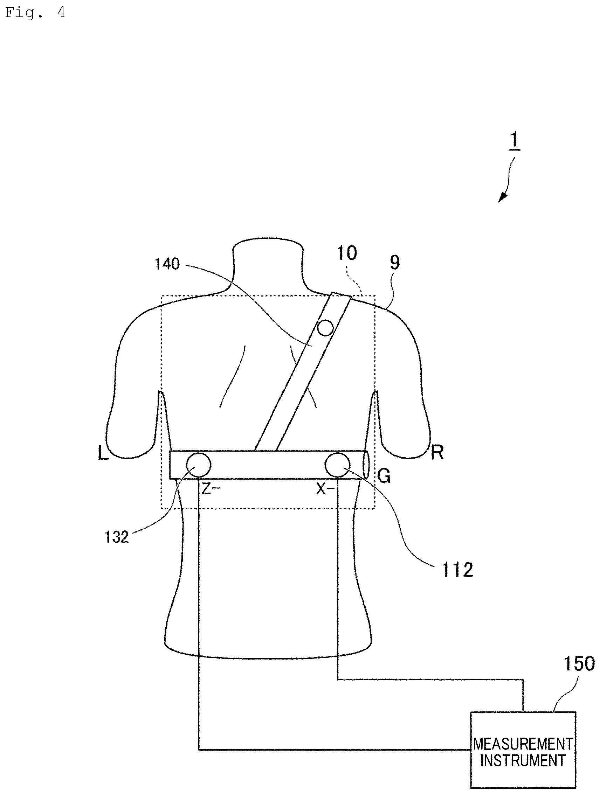

   Fig. 4 is a diagram showing an example of a view of the upper half of the body of the wearer 9 wearing the lead instrument 1 of the embodiment when viewed from a back.
[Fig. 5]

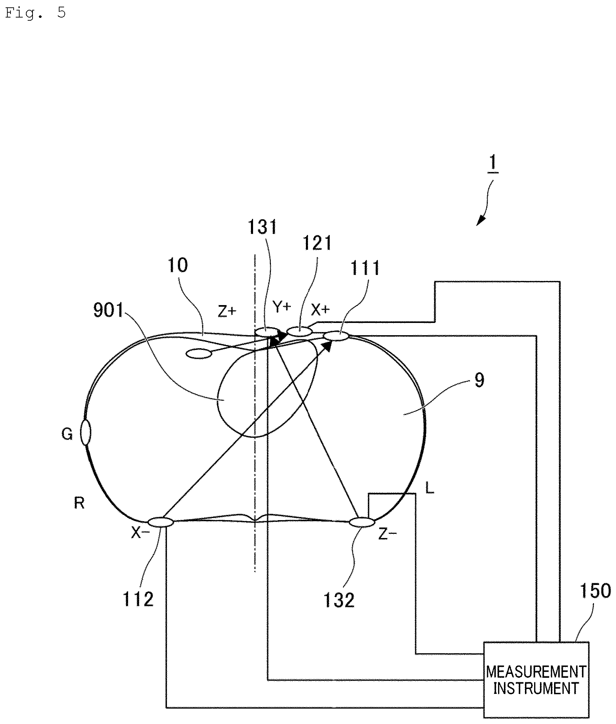

   Fig. 5 is a diagram showing an example of a cross-sectional view of the wearer 9 wearing the lead instrument 1 of the embodiment.
[Fig. 6]

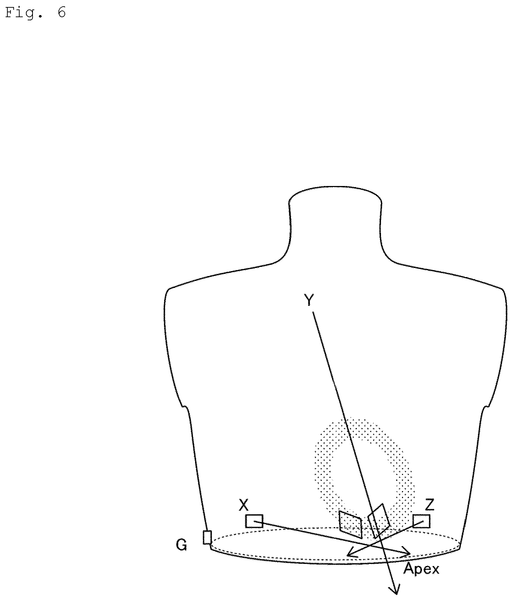

   Fig. 6 is a first diagram showing an example of a positional relationship between the lead instrument 1 and the heart 901 of the wearer 9 of the embodiment.
[Fig. 7]

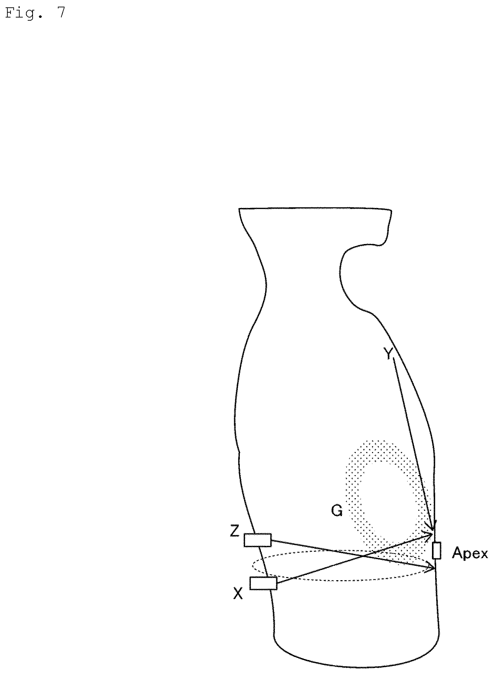

   Fig. 7 is a second diagram showing an example of the positional relationship between the lead instrument 1 and the heart 901 of the wearer 9 of the embodiment.
[Fig. 8]

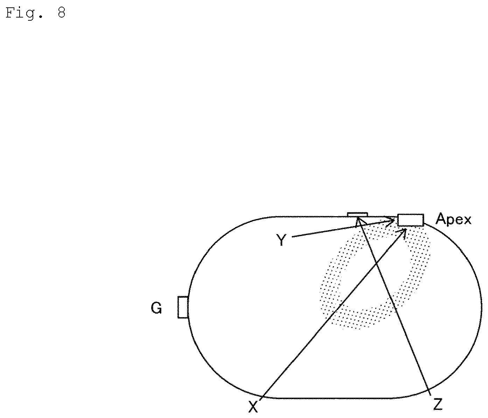

   Fig. 8 is a third diagram showing an example of the positional relationship between the lead instrument 1 and the heart 901 of the wearer 9 of the embodiment.
[Fig. 9]

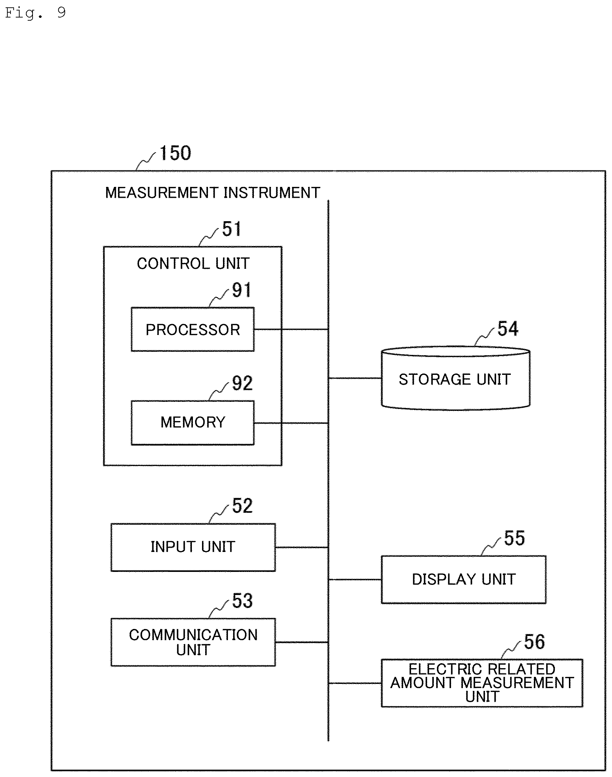

   Fig. 9 is a diagram showing an example of a hardware configuration of a measurement instrument 150 according to the embodiment.
[Fig. 10]

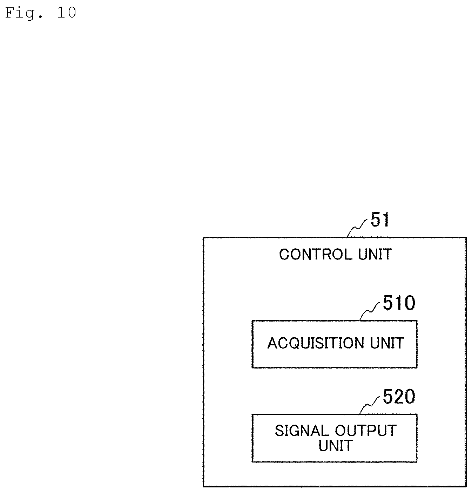

   Fig. 10 is a diagram showing an example of a functional configuration of a control unit 51 according to the embodiment.
[Fig. 11]

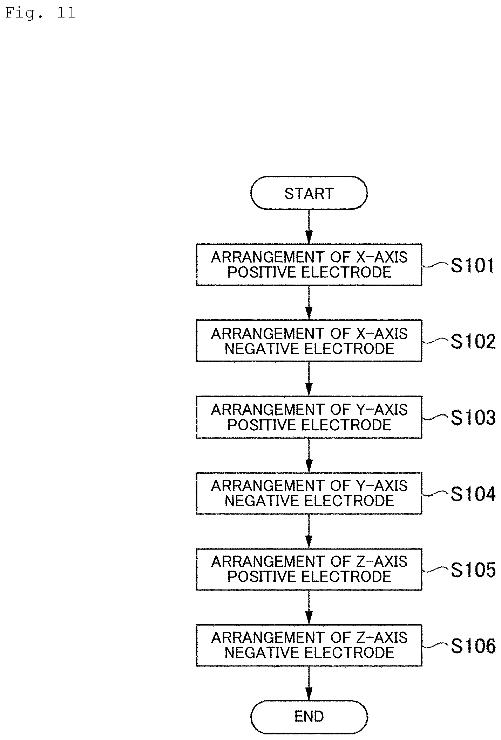

   Fig. 11 is a flowchart showing an example of a flow of processing executed by an electrode arrangement method which is the method for arranging the electrodes of the embodiment.
[Fig. 12]

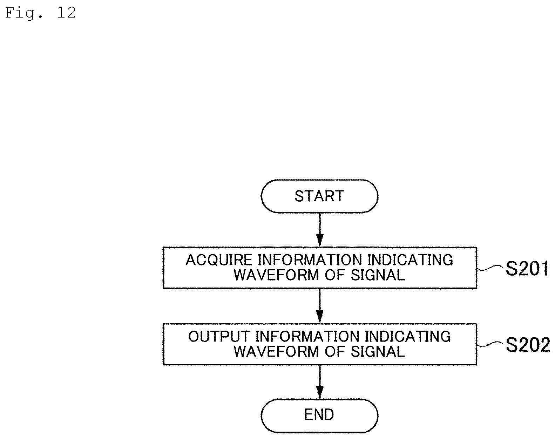

   Fig. 12 is a flowchart showing an example of a flow of processing executed by the lead instrument 1 according to the embodiment.
[Fig. 13]

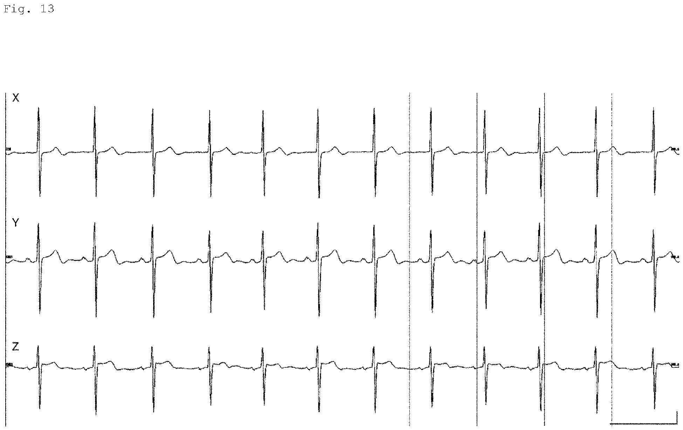

   Fig. 13 is a first diagram showing an example of the heart electrocardiogram of a healthy person acquired using the lead instrument 1 according to the embodiment.
[Fig. 14]

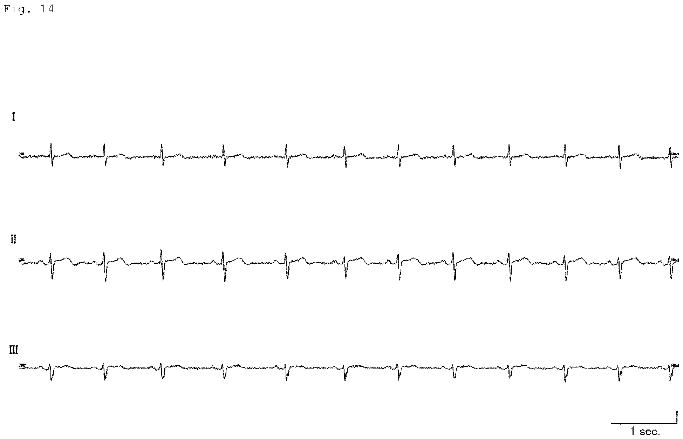

   Fig. 14 is a first diagram of the heart of the healthy person acquired using a standard electrocardiogram lead method to be compared.
[Fig. 15]

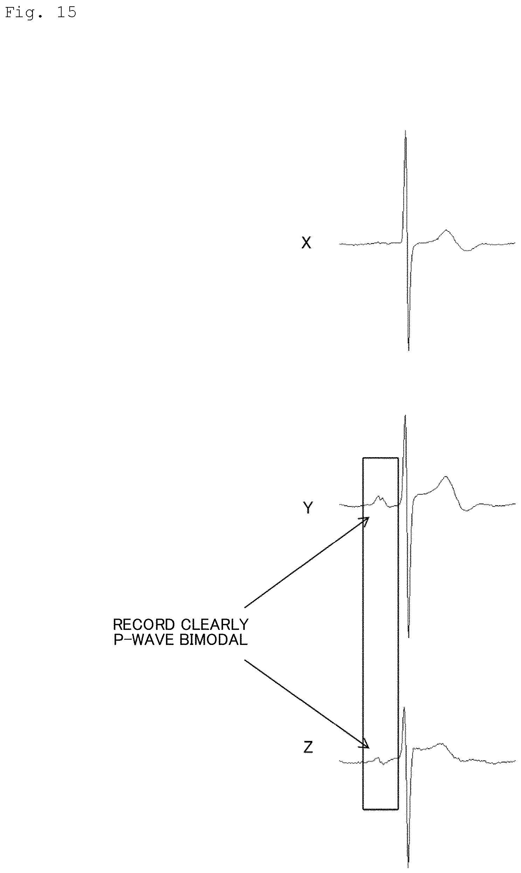

   Fig. 15 is a second diagram showing the electrocardiogram measured by using the lead instrument 1 according to the embodiment.
[Fig. 16]

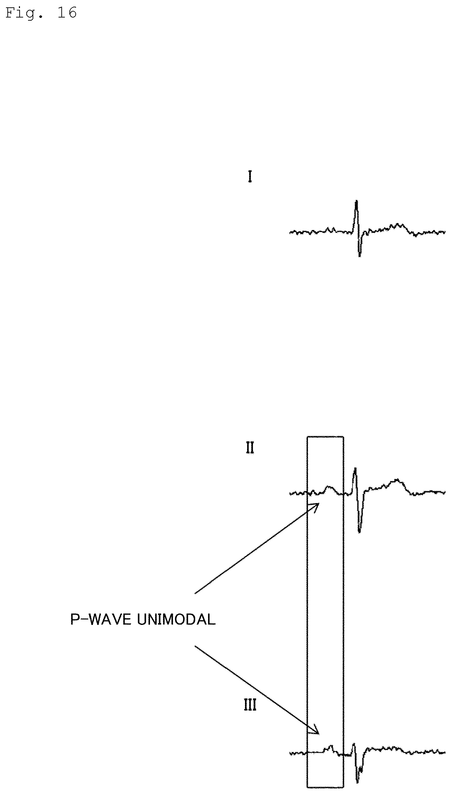

   Fig. 16 is a second diagram of the heart of the healthy person acquired using the standard electrocardiogram lead method to be compared.
[Fig. 17]

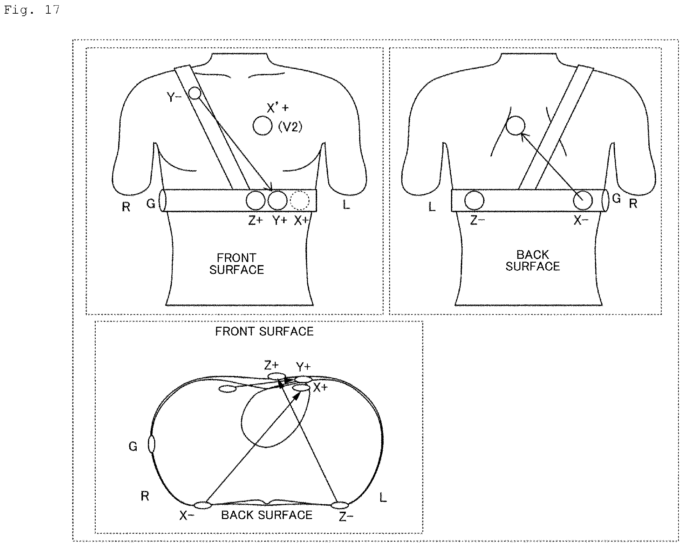

   Fig. 17 is a first diagram showing an example of the electrode arrangement according to a modification example.
[Fig. 18]

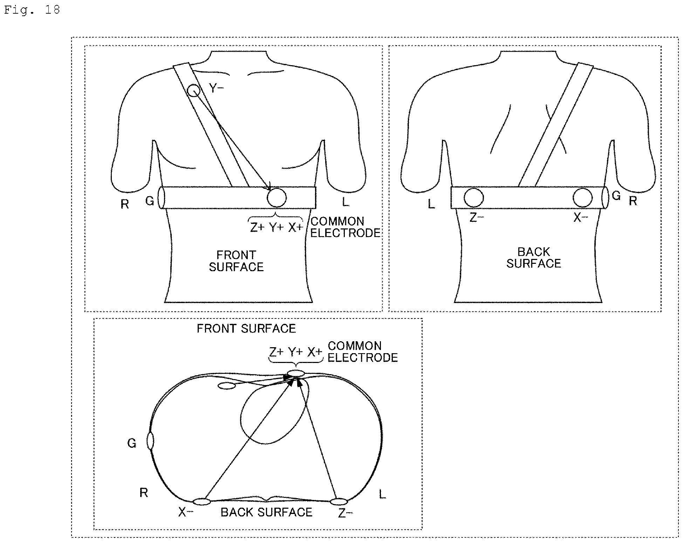

   Fig. 18 is a second diagram showing an example of the electrode arrangement according to a modification example.
[Fig. 19]

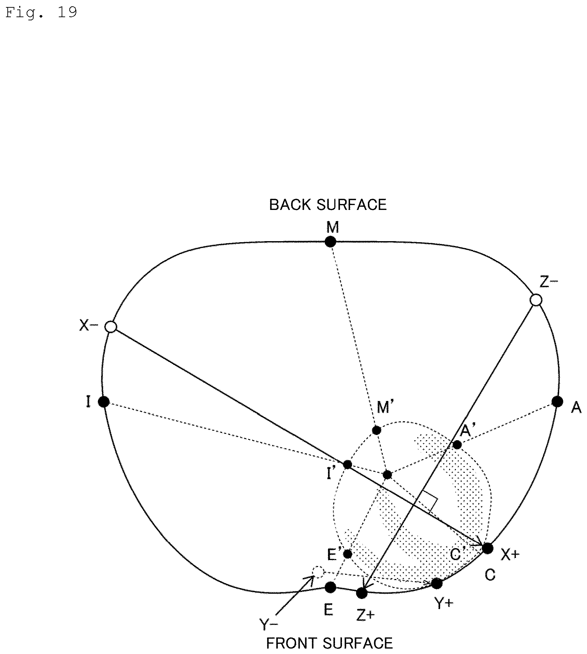

   Fig. 19 is a diagram showing an example of a horizontal cross-section of a chest at the height of a band body 140.
[Fig. 20]

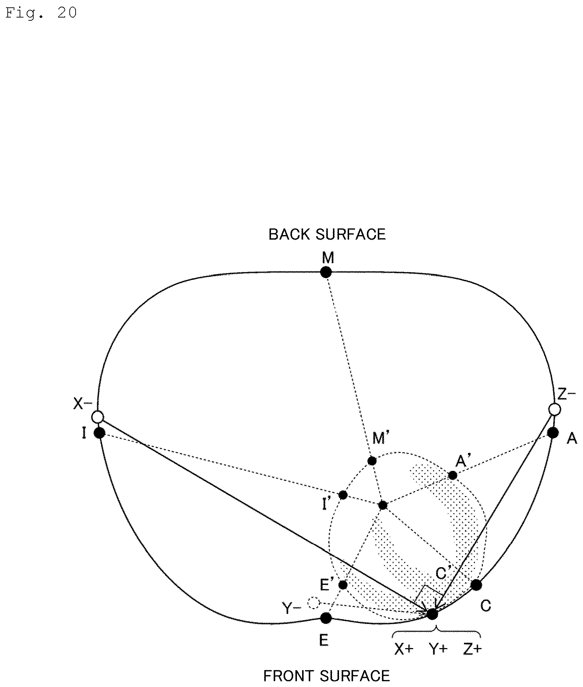

   Fig. 20 is a diagram of an explanation of a lead method of Frank.

### [Description of Embodiments]

### (Embodiment)

First, a relationship between information obtained from a waveform of an electrocardiogram and an arrangement of an electrodes when the waveform of the electrocardiogram is acquired will be described with reference to Figs. 1 and 2.

Fig. 1 is a first explanatory diagram for explaining the relationship between the information obtained from the waveform of the electrocardiogram and the arrangement of the electrodes when the waveform of the electrocardiogram is acquired. Fig. 2 is a second explanatory diagram for explaining the relationship between the information obtained from the waveform of the electrocardiogram and the arrangement of the electrodes when the waveform of the electrocardiogram is acquired. Figs. 1 and 2 show examples of an anatomical space and an image space corresponding to the anatomical space. The anatomical space is a real space. The image space is an image space described in Reference Document 1.
Reference Document 1: ERNAEST FRANK, "An Accurate, Clinically Practical Systen For Spatial Vectorcardiography", Circulation, Volume XIII, May, 1956, 737-749
Reference Document 2: Clinical Vectorcardiography 2nd Grune & Stratton Te-Chuan Chou 1974

One of the methods of representing information is a method of using vectors in a space in analytics and in a vector space in which an inner product is defined. In such a method, a base having the number of axes corresponding to the dimensions of the vector space is used for the expression of the information. For this reason, for example, if the vector space representing information is a three-dimensional space, a base having three axes is used for representing the information.

In view of a linear independence of the vector in the vector space, the amount of information represented by each point in the vector space is larger as the information represented by each axis of the base differs from the information represented by the other axes. Therefore, in the case where each axis of the base represents the observed information, it is desirable from the viewpoint of increasing the amount of information that the observation is performed so that each axis of the base is arranged close to a linear independent arrangement in the vector space.

The close linear independence means that orthogonality is high, and the high orthogonality means that the difference in the direction between the axes of the bases is large. More specifically, the high orthogonality means, for example, that the sum of the inner products of the axial vectors is large. The sum means to sum all of the base sets that are sets of one of the axes that the base has and another one of the axes that the base has. The axial vector inner product means an inner product of two different axial vectors. The axial vector is a unit vector parallel to the axis of the base.

Therefore, in order to increase the amount of information obtained from the waveform of the electrocardiogram, it is desirable to perform the observation so that the orthogonality of the respective axes of the base in the information space is higher. The information space means a vector space in analytics, the vector space in which the inner product is defined and the vector space which represents the information. Hereinafter, the base in the information space is referred to as an information base. Hereinafter, each axis of the information base is referred to as an information axis.

One or a plurality of electrode pairs, which is a pair of an anode and a cathode, are used for acquiring the waveform of the electrocardiogram. Then, in the acquisition of the waveform of the electrocardiogram, the information obtained from one electrode pair is the information represented by one information axis. The information obtained from one electrode pair is specifically information of a signal propagating on the straight line (referred to as "observation axis" below) connecting an anode and a cathode of the electrode pair.

In Reference Document 1, a method for obtaining an arrangement of the information axis in an electrophysiological image space of a human from an arrangement of the observation axis in an anatomical space, (hereinafter referred to as a "Frank method") is described. By using the Frank method, an arrangement having higher orthogonality in the image space is obtained among candidates for the arrangement of electrodes actually possible in the anatomical space.

An X-axis, a Y-axis and a Z-axis in the anatomical space of Fig. 1 are observation axes, respectively. Note that, in Fig. 1, the X-axis is a straight line connecting X- and X+. Note that, in Fig. 1, the Y-axis is a straight line connecting Y-and Y+. Note that, in Fig. 1, the Z-axis is a straight line connecting Z- and Z+.

The X-axis, Y-axis and Z-axis in the image space of Fig. 1 are information axes obtained by the Frank method, respectively, and are information axes in the image space corresponding to the observation axis of the anatomical space. Hereinafter, the X-axis of the observation axis is referred to as an X observation axis. Hereinafter, the Y-axis of the observation axis is referred to as a Y observation axis. Hereinafter, the Z-axis of the observation axis is referred to as a Z observation axis. Hereinafter, the X-axis of the information axis is referred to as an X-information axis. Hereinafter, the Y-axis of the information axis is referred to as a Y-information axis. Hereinafter, the Z-axis of the information axis is referred to as a Z-information axis. When viewed from the viewpoint of orthogonality, the three information axes of Figs. 1 and 2 are an example of arrangement having high orthogonality.

Figs. 1 and 2 show that the X and Z observation axes pass through a cardiac apex part or a ventricle anterior wall. The ventricle anterior wall is a ventricular wall in contact with the anterior chest wall. In practice, in order to increase the amount of information obtained from the waveform of the electrocardiogram, it is desirable that the observation axis passes through the cardiac apex part or the ventricle anterior wall from a viewpoint different from the orthogonality of the information axis. The part from cardiac apex part to the ventricle anterior wall is close to the anterior chest wall, and a distinct cardiac potential with a large amplitude can be recorded. In general, when a hand is attached to a vicinity of the heart, pulsation of the heart can be felt, but actually, the pulsation can be felt strongest when the hand is attached to the vicinity of the cardiac apex part of the heart or the ventricle anterior wall. In this way, the ventricular muscle near the cardiac apex part is close to the chest wall and the pulsation of the heart appears strongly. The region of the ventricular muscle in the vicinity of the cardiac apex part corresponds to, for example, 13 Apical Anterior in a 16-division left ventricular model, and further includes 14 Apical Septal, 15 Apical Inferior, and 16 Apical Lateral. Further, 7 Mid Anterior 12 Mid Anterolateral may be included. In the case of the 17-division method, 17 Apex is added to the above-mentioned region.

Since it is desirable that the intensity of the signal of information source is stronger than noise in obtaining information, it is desirable that the intensity of the signal of the information source is stronger than noise, and in viewpoint of obtaining information from the waveform of the electrocardiogram, it is desirable that the potential of the cardiac apex part or the ventricle anterior wall (called "the vicinity of the cardiac apex part" below) is obtained as the waveform of the electrocardiogram. Therefore, in order to increase the information obtained from the waveform of the electrocardiogram, the X and Z observation axes in Figs. 1 and 2 pass through the vicinity of the cardiac apex part.

Figs. 1 and 2 show that the Y observation axis is substantially parallel to the main axis of the electromotive force vector of the heart. The main axis of the electromotive force vector of the heart is, for example, the maximum vector between QRSs in a vector electrocardiogram and is substantially parallel to the standard II guidance of a normal scalar electrocardiogram. Indeed, it is medically known that in order to increase the information obtained from the waveform of the electrocardiogram, the more the at least one of the observation axes is parallel to the main axis of the electromotive force vector of the heart, the more distinct cardiac potential with a larger amplitude can be recorded, and the more information amount is present. Therefore, in Figs. 1 and 2, the Y observation axis is parallel to the main axis of the electromotive force vector of the heart.

For these reasons, the electrodes are arranged so as to form the observation axis as the arrangement shown in Figs. 1 and 2, thereby increasing the amount of information obtained from the waveform of the electrocardiogram. More specifically, when the arrangement of the electrodes satisfies the X observation axis condition, the Y observation axis condition, the Z observation axis condition and the orthogonality condition, the amount of information obtained from the waveform of the electrocardiogram increases.

The X observation axis condition includes a condition that the X observation axis passes through the vicinity of the cardiac apex part of the wearer 9, and an axis connecting the left front chest and the right back chest. The Y observation axis condition is a condition that the Y observation axis is substantially parallel to the main axis of the electromotive force vector of the heart. The Z observation axis condition includes a condition that the Z observation axis is non-parallel to the X observation axis and a condition that the Z observation axis passes through the vicinity of the cardiac apex part of the wearer 9. The condition of the Y observation axis may also be such that the Y observation axis passes through the vicinity of the cardiac apex part, in addition to the condition that the Y observation axis is substantially parallel to the main axis of the electromotive force vector of the heart.

The orthogonality condition is a condition that the arrangement of the observation axes satisfies a condition that the height of orthogonality is higher than a predetermined height in the image space. That is, the orthogonality condition is a condition that orthogonality of bases with three axes of the X information axis, the Y information axis and the Z information axis as axes is higher than the predetermined height. The predetermined height is a height corresponding to the amount of information that the user wants to obtain from the waveform of the electrocardiogram. As the predetermined height is higher, the amount of information obtained for the user from the waveform of the electrocardiogram is larger. The arrangement of the X information axis, the Y information axis, and the Z information axis satisfying the orthogonality condition is, for example, the arrangement in which the X information axis, the Y information axis, and the Z information axis are substantially orthogonal. Although it is not always realized in accordance with the state of the body position of the wearer 9 or the like, it is desirable that the X information axis, the Y information axis and the Z information axis are substantially orthogonal because the higher the orthogonality is, the larger the amount of information obtained from the waveform of the electrocardiogram for the user in consideration from viewpoint of the obtained amount of information.

Therefore, the X observation axis condition is, for example, a condition that the X observation axis is parallel to a vector from the back of the wearer 9 to the cardiac apex part of the wearer 9. The Z observation axis condition is, for example, a condition that the Z observation axis is not parallel to the X observation axis and the Z observation axis is parallel to a vector from the back of the wearer 9 to the cardiac apex part of the wearer 9.

An example of the lead instrument 1 of the embodiment will be described with reference to Figs. 3 to 8. Fig. 3 is a diagram showing an example of the upper half of a body of the wearer 9 wearing the lead instrument 1 of the embodiment viewed from the front. Fig. 4 is a diagram showing an example of the upper half of the body of the wearer 9 wearing the lead instrument 1 of the embodiment viewed from the back. Fig. 5 is a diagram showing an example of the cross-sectional view of the wearer 9 wearing the lead instrument 1 of the embodiment.

Fig. 6 is a first diagram showing an example of the positional relationship between the lead instrument 1 and the heart 901 of the wearer 9 of the embodiment. Fig. 7 is a second diagram showing an example of the positional relationship between the lead instrument 1 and the heart 901 of the wearer 9 of the embodiment. Fig. 8 is a third diagram showing an example of the positional relationship between the lead instrument 1 and the heart 901 of the wearer 9 of the embodiment.

For the sake of simplicity, one of a pair of electrodes is represented as a positive electrode and the other is represented as a negative electrode, but the positive electrode and the negative electrode may be arranged at opposite positions. When the positions of the positive electrode and the negative electrode are opposite to each other, positive and negative potentials in the waveform of the electrocardiogram are expressed in opposite directions.

The lead instrument 1 acquires the waveform of the electrocardiogram of the wearer 9 by a bipolar lead method. The lead instrument 1 includes six electrodes of an X-axis positive electrode 111, an X-axis negative electrode 112, a Y-axis positive electrode 121, a Y-axis negative electrode 122, a Z-axis positive electrode 131 and a Z-axis negative electrode 132, a band body 140, and a measurement instrument 150.

The X-axis positive electrode 111 is an electrode located at an intersection between the X observation axis and the body surface of the front of the wearer 9. The X-axis negative electrode 112 is an electrode located at an intersection between the X observation axis and the body surface of the back of the wearer 9.

The Y-axis positive electrode 121 is an electrode located at a position close to the lower half of the body at an intersection between the Y observation axis and the body surface of the wearer 9. The Y-axis negative electrode 122 is an electrode located at a position farther from the lower half of the body than the Y-axis positive electrode 121 out of intersections between the Y observation axis and the body surface of the wearer 9.

The Z-axis positive electrode 131 is an electrode located at an intersection between the Z observation axis and the body surface of the front of the wearer 9. The Z-axis negative electrode 132 is an electrode located at an intersection between the Z observation axis and the body surface of the back of the wearer 9.

The arrangement of the X observation axis, the Y observation axis and the Z observation axis satisfies the X observation axis condition, the Y observation axis condition, the Z observation axis condition and the orthogonality condition.

The band body 140 fixes the X-axis positive electrode 111, the X-axis negative electrode 112, the Y-axis positive electrode 121, the Y-axis negative electrode 122, the Z-axis positive electrode 131 and the Z-axis negative electrode 132 to the wearer 9. A band 10 is formed by the band body 140 and the X-axis positive electrode 111, the X-axis negative electrode 112, the Y-axis positive electrode 121, the Y-axis negative electrode 122, the Z-axis positive electrode 131 and the Z-axis negative electrode 132. That is, the band 10 includes the X-axis positive electrode 111, the X-axis negative electrode 112, the Y-axis positive electrode 121, the Y-axis negative electrode 122, the Z-axis positive electrode 131, the Z-axis negative electrode 132, and the band body 140.

The band body 140 is, for example, composed of a belt for shoulder (referred to "a shoulder belt" below) and a belt wound around the body surface of the wearer 9 in a cross section including the vicinity of the cardiac apex part (referred to "a lateral belt" below). The shoulder belt includes, for example, the Y-axis negative electrode 122, and the Y-axis negative electrode 122 is fixed to the body surface of the wearer 9. The lateral belt includes, for example, the electrodes other than the Y-axis negative electrode 122, and the electrodes other than the Y-axis negative electrode 122 are fixed to the body surface of the wearer 9.

The measurement instrument 150 acquires signals propagating through the X observation axis, the Y observation axis and the Z observation axis. The waveform of the signal acquired by the measurement instrument 150 is the waveform of the electrocardiogram. Specifically, the measurement instrument 150 measures a current value or a voltage of a current flowing through each of the X observation axis, the Y observation axis, and the Z observation axis to acquire the signals propagating through each of the observation axes. For this purpose, the measurement instrument 150 is a device having three ammeters, for example, an ammeter for measuring the current value of the current flowing through the X observation axis, an ammeter for measuring the current value of the current flowing through the Y observation axis, and an ammeter for measuring the current value of the current flowing through the Z observation axis.

Fig. 9 is a diagram showing an example of a hardware configuration of the measurement instrument 150 according to the embodiment. The measurement instrument 150 includes a control unit 51 including a processor 91 such as a CPU (Central Processing Unit) and a memory 92, which are connected by a bus, and executes a program. The measurement instrument 150 functions as the device including the control unit 51, an input unit 52, a communication unit 53, a storage unit 54, a display unit 55, and an electric related amount measurement unit 56 by executing the program. More specifically, the processor 91 reads the program stored in the storage unit 54 and stores the read program in the memory 92. As a result of the processor 91 executing the program stored in the memory 92, the measurement instrument 150 functions as the device including the control unit 51, the input unit 52, the communication unit 53, the storage unit 54, the display unit 55, and the electric related amount measurement unit 56.

The control unit 51 controls operations of the functional units included in the measurement instrument 150. The control unit 51 controls, for example, the operation of the electric related amount measurement unit 56.

The input unit 52 is configured to include an input device such as a mouse, a keyboard, and a touch panel. The input unit 52 may be configured as an interface for connecting any of these input devices to the measurement instrument 150. The input unit 52 accepts input of various kinds of information to the measurement instrument 150. The input unit 52 accepts input of an instruction for starting the operation, for example.

The communication unit 53 is configured to include a communication interface for connecting the measurement instrument 150 to an external device. The communication unit 53 performs communication with external device which is the connected communication destination capable of the wired communication or the wireless communication. The communication unit 53 exchanges information with the external device by communication with the external device.

The storage unit 54 is configured by using a non-transitory computer-readable storage medium device such as a magnetic hard disk device or a semiconductor storage device. The storage unit 54 stores various kinds of information relating to the measurement instrument 150. The storage unit 54 stores, for example, the information inputted via the input unit 52 or the communication unit 53. The storage unit 54 stores, for example, the information acquired by the electric related amount measurement unit 56.

The display unit 55 displays various kinds of information. The display unit 55 is configured to include a display device such as a CRT (Cathode Ray Tube) display, a liquid crystal display, or an organic EL (Electro-Luminescence) display, for example. The display unit 55 may be configured as an interface for connecting any of these display devices to the measurement instrument 150. The display unit 55 outputs the information that is inputted to the input unit 52, for example.

The electric related amount measurement unit 56 acquires the information indicating the waveforms of the signals propagating through the X observation axis, the Y observation axis and the Z observation axis. The electric related amount measurement unit 56 is a voltmeter or an ammeter connected to, for example, the X-axis positive electrode 111, the X-axis negative electrode 112, the Y-axis positive electrode 121, the Y-axis negative electrode 122, the Z-axis positive electrode 131 and the Z-axis negative electrode 132. The electric related amount measurement unit 56 records the information indicating the waveform of the acquired signal in the storage unit 54.

The acquisition of the information indicating the waveform of the signal means that the electric related amount measurement unit 56 measures the potential or current of the signal at each time during the measurement period. The time series of the measurement results is an example of the information indicating the waveform of the signal. Therefore, the information indicating the waveform of the signal is, for example, the electrocardiogram.

Fig. 10 is a diagram showing an example of a functional configuration of the control unit 51 in the embodiment. The control unit 51 includes an acquisition unit 510, and a signal output unit 520.

The acquisition unit 510 controls the operation of the electric related amount measurement unit 56 to acquire the information indicating waveforms of signals propagating through the X observation axis, the Y observation axis, and the Z observation axis.

The signal output unit 520 outputs the information indicating the waveform of the signal obtained by the acquisition unit 510 to a predetermined output destination. The predetermined output destination is, for example, the storage unit 54. In such a case, the storage unit 54 stores the information indicating the waveform of the signal obtained by the acquisition unit 510. The predetermined output destination may be any output destination as long as it is the predetermined output destination.

The predetermined output destination is, for example, the display unit 55. In such a case, the information indicating the waveform of the signal obtained by the acquisition unit 510 is displayed on the display unit 55. The predetermined output destination is, for example, a storage device communicably connected to the communication unit 53. In such a case, the information indicating the waveform of the signal obtained by the acquisition unit 510 is recorded in the storage device communicably connected to the communication unit 53. The storage device communicably connected to the communication unit 53 is an example of the external device. The storage device communicably connected to the communication unit 53 is, for example, a memory card.

Fig. 11 is a flowchart showing an example of a flow of processing executed by the electrode arrangement method which is the method for arranging the electrodes of the embodiment. The X-axis positive electrode 111 is arranged on the body surface of the wearer 9 (step S101). Next, the X-axis negative electrode 112 is arranged on the body surface of the wearer 9 (step S102). That is, the X-axis negative electrode 112 is arranged on the body surface of the wearer 9 so that the X observation axis passes through the cardiac apex part of the wearer 9. Next, the Y-axis positive electrode 121 is arranged on the body surface of the wearer 9 (step S103). Next, the Y-axis negative electrode 122 is arranged on the body surface of the wearer 9 (step S104). Next, the Z-axis positive electrode 131 is arranged on the body surface of the wearer 9 (step S105). Next, the Z-axis negative electrode 132 is arranged on the body surface of the wearer 9 (step S106).

In the electrode arrangement method, the processing from step S101 to step S106 do not necessarily have to be executed in the order shown in Fig. 11. The processing of the step S101 to step S106 may be executed in any order if it is executed during the execution of the electrode arrangement method, or a part or all of the processing of the step S101 to step S106 may be executed simultaneously. After the step S102, step S104 and step S106, the cardiac potentials of the X-axis, the Y-axis and the Z-axis are confirmed, for example, the adjustment of the arrangement and the confirmation of the contact state of the electrodes may be executed from a wave height, noise and SN ratio of the R wave and the P-wave.

Fig. 12 is a flowchart showing an example of the flow of processing executed by the lead instrument 1 according to the embodiment. The acquisition unit 510 acquires the information indicating waveforms of signals propagating through the X observation axis, the Y observation axis and the Z observation axis (step S201). Next, the signal output unit 520 outputs the information indicating the waveform of the signal obtained in the step S201 to the predetermined output destination (step S202).

The lead instrument 1 does not necessarily have to include the measurement instrument 150. The current value or voltage of the current flowing through each of the X-observation axis, the Y-observation axis and the Z-observation axis may be measured by an external device capable of measuring the current value or voltage of the current flowing through each of the X-observation axis, the Y-observation axis and the Z-observation axis, then the lead instrument 1 does not necessarily have to include the measurement instrument 150.

Fig. 13 is a first diagram showing an example of an electrocardiogram of the heart of a healthy person acquired using the lead instrument 1 according to the embodiment. Fig. 13 shows three graphs, namely, a graph showing the waveform of the signal propagating on the X observation axis, a graph showing the waveform of the signal propagating on the Y observation axis, and a graph showing the waveform of the signal propagating on the Z observation axis. In each graph, a horizontal axis indicates a time point and a vertical axis indicates a potential. The scale bars are 1 second and 0.5 mV, respectively.

Fig. 14 is a first diagram of the heart of the healthy person obtained by using a standard electrocardiogram lead method to be compared. In the standard electrocardiogram lead method, the same measurement instrument as the measurement instrument 150 and the electrodes which are electrodes of the same material and shape as the X-axis positive electrode 111, the X-axis negative electrode 112, the Y-axis positive electrode 121, the Y-axis negative electrode 122, the Z-axis positive electrode 131 and the Z-axis negative electrode 132 and have different attachment destinations are used. In Fig. 14, a healthy subject is the same subject as the subject shown in Fig. 13. Thus, the results shown in Fig. 14 are the electrocardiograms of the healthy person obtained by the conventional standard limb lead. I represents an upper right limb and an upper left limb, II represents an upper right limb and a lower left limb, and III represents the potential between the upper left limb and the lower left limb. In each graph, a horizontal axis indicates a time point and a vertical axis indicates a potential. The scale bars are 1 second and 0.5 mV, respectively.

Fig. 13 shows that the waves, such as PQRSTU, are clearly separated from each other, and the inflection point and phase are different in the XYZ axes as compared with Fig. 14. Fig. 13 shows that such characteristics appear on the P-wave and the ST wave. Fig. 13 shows that a lot of information is shown in the electrocardiogram of the heart of the healthy person obtained by using the lead instrument 1. That is, Fig. 13 shows that an electrocardiogram rich in information quantity with a good SN ratio is obtained by using the lead instrument 1. Note that the rich amount of information specifically means that each wave, such as PQRSTU, is clearly separated, and the inflection point and phase are different in the XYZ axis, and in particular the characteristics appear on the P-wave and the ST wave.

Fig. 15 is a second diagram showing an example of the electrocardiogram measured by using the lead instrument 1 according to the embodiment. More specifically, Fig. 15 is a diagram showing a partially enlarged graph of Fig. 13.

Fig. 16 is a second diagram of the heart of the healthy person obtained by using the standard electrocardiogram lead method to be compared. More specifically, Fig. 16 is a diagram showing a partially enlarged graph of Fig. 14. In the standard electrocardiogram lead method, the same measurement instrument as the measurement instrument 150 and the electrodes which are electrodes of the same material and shape as the X-axis positive electrode 111, the X-axis negative electrode 112, the Y-axis positive electrode 121, the Y-axis negative electrode 122, the Z-axis positive electrode 131 and the Z-axis negative electrode 132 and have different attachment destinations are used. In Fig. 16, a healthy subject is the same subject as the subject shown in Fig. 15. Thus, the results shown in Fig. 16 are the electrocardiograms of the healthy person obtained by the conventional standard limb lead.

Fig. 15 shows that P-wave bimodal characteristics appear in a graph showing the waveform of the signal propagating on the Y observation axis and a graph showing the waveform of the signal propagating on the Z observation axis. Fig. 15 shows that excitations of the right atrium and the left atrium of the P-wave are clearly recorded in the graph showing the waveform of the signal propagating on the Y observation axis and the graph showing the waveform of the signal propagating on the Z observation axis. On the other hand, the P-wave of Fig. 16 by the standard limb lead is unimodal, and the information on the excitations of the right atrium and left atrium is not recorded.

The lead instrument 1 configured in this way acquires the signal propagating through the straight line passing through the vicinity of the cardiac apex part by the bipolar lead using the electrode located at the intersection between the straight line passing through the vicinity of the cardiac apex part and the body surface of the wearer 9. Since the intensity of the signal is strong in the vicinity of the cardiac apex part, the lead instrument 1 can increase the information obtained from the waveform of the electrocardiogram.

The lead instrument 1 thus configured acquires the signal propagating through the observation axis arranged so as to be arranged with high orthogonality in the image space. Therefore, the lead instrument 1 can increase the information obtained from the waveform of the electrocardiogram.

### (Modification Example)

Note that the X observation axis, the Y observation axis, and the Z observation axis does not always need to satisfy all of the X observation axis conditions, the Y observation axis conditions, the Z observation axis condition and the orthogonality condition. The X observation axis, the Y observation axis and the Z observation axis may satisfy only the orthogonality condition, for example. The X observation axis, the Y observation axis and the Z observation axis may satisfy only the X observation axis condition, for example. The X observation axis, the Y observation axis and the Z observation axis may satisfy only the Z observation axis condition, for example.

It is not always necessary for the lead instrument 1 to have six electrodes. The lead instrument 1 may include, for example, the X-axis positive electrode 111 and the X-axis negative electrode 112, the Z-axis positive electrode 131 and the Z-axis negative electrode 132, and may not include the Y-axis positive electrode 121 and the Y-axis negative electrode 122.

The lead instrument 1 may include seven or more electrodes including, for example, the X-axis positive electrode 111 and the X-axis negative electrode 112, and the Z-axis positive electrode 131 and the Z-axis negative electrode 132.

The lead instrument 1 may acquire only the signal propagating through the X observation axis satisfying, for example, the X observation axis condition, and in this case, the lead instrument 1 may include only the X-axis positive electrode 111 and the X-axis negative electrode 112. The lead instrument 1 may include seven or more electrodes, for example, including an X-axis positive electrode 111 and an X-axis negative electrode 112.

The lead instrument 1 may acquire only the signal propagating through the Z observation axis satisfying, for example, the Z observation axis condition, and in this case, the lead instrument 1 may include only the Z-axis positive electrode 131 and the Z-axis negative electrode 132. The lead instrument 1 may include seven or more electrodes, for example, including the Z-axis positive electrode 131 and the Z-axis negative electrode 132.

Fig. 17 is a first diagram showing an example of the electrode arrangement according to the modification example. In Fig. 17, X'+ represents a new position of the X-axis positive electrode 111 according to the modification example, and X+ represent the original position of the X-axis positive electrode 111. X- represents the X-axis negative electrode 112. In Fig. 17, Y+ represents the Y-axis positive electrode 121 and Y- represents the Y-axis negative electrode 122. In Fig. 17, Z+ represents the Z-axis positive electrode 131 and Z- represents the Z-axis negative electrode 132. In Fig. 17, the X-axis positive electrode 111 is located at the left edge of the fourth intercostal sternum. That is, the X observation axis is a straight line extending from the back of the wearer 9 to the left edge of the fourth intercostal sternum. In Fig. 17, the X observation axis is not parallel to the Z observation axis and is not parallel to the Y observation axis. Since the left edge of the fourth intercostal sternum is in the vicinity of the pulmonary artery outflow path and is a portion where abnormal early repolarization of a myocardium in Brugada syndrome is likely to occur, in the case of the arrangement shown in Fig. 17, the abnormality of the outflow path in Brugada syndrome can be detected.

Fig. 18 is a second diagram showing an example of the electrode arrangement according to the modification example. In Fig. 18, X+ represents the X-axis positive electrode 111, and X- represents the X-axis negative electrode 112. In Fig. 18, Y+ represents the Y-axis positive electrode 121 and Y-represents the Y-axis negative electrode 122. In Fig. 18, Z+ represents the Z-axis positive electrode 131 and Z- represents the Z-axis negative electrode 132. Fig. 18 shows that the X-axis positive electrode 111, the Y-axis positive electrode 121 and the Z-axis positive electrode 131 are the same electrode. In this modification example, the number of electrodes and wires can be reduced by using one electrode in the vicinity of the cardiac apex part. In the case of the narrow anterior chest part such as a petite subject and a child, an installation of the electrode is easy.

Note that the X-axis positive electrode 111 is an example of a first-axis positive electrode. The X-axis negative electrode 112 is an example of a first-axis negative electrode. The X observation axis is an example of a first observation axis. The Y observation axis is an example of a third observation axis. The Z observation axis is an example of a second observation axis. The Z-axis positive electrode 131 is an example of a second-axis positive electrode. The Z-axis negative electrode 132 is an example of a second-axis negative electrode. The Y-axis positive electrode 121 is an example of a third-axis positive electrode. The Y-axis negative electrode 122 is an example of a third-axis negative electrode. The band 10 is an example of an attachment tool. The band body 140 is an example of a body of the attachment tool.

Fig. 19 shows an example in detail. More specifically, Fig. 19 is a diagram showing an example of a horizontal cross section of the chest at the height of the band body 140. In Fig. 19, the upper is the back side and the lower is the anterior chest part. Pints of A, C, E, I, M and a solid line connecting them indicates the coordinates of the electrodes on the anatomical horizontal cross-section of the chest of a lead method of Frank. A dotted line connecting points of A', C', E', I', and M' indicates the chest horizontal cross section of the image space of Frank, and points of A', C', E', I', and M' indicate the coordinates of the electrodes corresponding to the anatomical coordinates of A, C, E, I, and M. The X-axis positive electrode is located at C in Fig., and the X-axis negative electrode located between the right back part I and M on the opposite side. The Z-axis positive electrode is located near the middle of the left anterior chest, and the Z-axis negative electrode is located on the opposite left back side. The X-axis and the Z-axis are orthogonal in the heart, and both anatomical and image space coordinates are linear independent. The Y-axis positive electrode is located between the X-axis positive electrode and the Z-axis positive electrode. The Y-axis negative electrode is installed at the right edge of the right subclavian sternum and constitutes a vertical Y-axis (a dotted line).

Fig. 20 shows an example. More specifically, Fig. 20 is a diagram for explaining the lead method of Frank. This example, like Fig. 19, indicates the anatomical coordinates (points of A, C, E, I and M on the solid line drawing the horizontal cross-section of the trunk) the coordinates in the image space (points of A', C', E', I' and M' on the dotted line drawing the horizontal cross-section of the image space). An example in which the X-axis positive electrode, the Y-axis positive electrode, and the Z-axis positive electrode are coupled to one point near the cardiac apex part is shown. The X-axis negative electrode takes a position close to I between I and M of the right back part on the opposite side, and the Z-axis negative electrode takes a position close to the left back side A. The X-axis and the Z-axis are orthogonal to each other at an intersection, and both anatomical and image space coordinates are linear independent. The Y-axis negative electrode is installed at the right edge of the right subclavian sternum and constitutes a vertical Y-axis (a dotted line).

Note that all or part of each function of the measurement instrument 150 may be implemented by using hardware such as an ASIC (Application Specific Integrated Circuit), a PLD (Programmable Logic Device), or an FPGA (Field Programmable Gate Array). A program may be recorded in a computer-readable recording medium. The computer-readable recording medium is, for example, portable medium such as a flexible disk, a magneto-optical disk, a ROM, a CD-ROM and a storage device such as a hard disk built into a computer system. The program may be transmitted via telecommunication lines.

Although the embodiments of the present invention have been described in detail with reference to the drawings, specific configurations are not limited to these embodiments, and the invention is defined by the appended claims.

### [Reference Signs List]

- 1: Lead instrument
- 10: Band
- 111: X-axis positive electrode
- 112: X-axis negative electrode
- 121: Y-axis positive electrode
- 122: Y-axis negative electrode
- 131: Z-axis positive electrode
- 132: Z-axis negative electrode
- 140: Band body
- 150: Measurement instrument
- 51: Control unit
- 52: Input unit
- 53: Communication unit
- 54: Storage unit
- 55: Display unit
- 56: Electric related amount measurement unit
- 510: Acquisition unit
- 520: Signal output unit
- 91: Processor
- 92: Memory

## Claims

1. A method for acquiring cardiac signals, comprising:
- arranging a first-axis positive electrode (111) and a first-axis negative electrode (112) on the surface of a wearer's body (9), such that a first observation axis, being a straight line connecting the first-axis positive electrode and the first-axis negative electrode, passes through a cardiac apex or an anterior wall of the ventricle of the wearer (9),
- arranging a second-axis positive electrode (131) and a second-axis negative electrode (132) on the body surface of the wearer, said second-axis positive electrode (131) being an electrode located at one of the intersections of a second observation axis passing through the cardiac apex part or the ventricle anterior wall of the wearer (9) and the body surface of the wearer, said second observation axis being a non-parallel straight line to the first observation axis; and said second-axis negative electrode (132) being an electrode located at the other one of the intersections of the second observation axis and the body surface of the wearer,
- arranging a third-axis positive electrode (121) and third-axis negative electrode (122) on the body surface of the wearer, such that a third observation axis which is a straight line connecting the third-axis positive electrode and the third-axis negative electrode is a non-parallel straight line to both the first observation axis and the second observation axis, and is substantially parallel to the electromotive force vector of the heart,
wherein the pair of the first-axis positive electrode (111) and the first-axis negative electrode (112), the pair of the second-axis positive electrode (131) and the second-axis negative electrode (132), and the pair of the third-axis positive electrode (121) and the third-axis negative electrode (122) are connected via a measuring device (150), and
- wherein a control unit is configured to acquire waveform information of signals propagating along the first, second, and third observation axes, and is configured to output the acquired information to a predetermined output destination.

2. The method according to claim 1, wherein the arrangement of the first observation axis, the second observation axis, and the third observation axis satisfies a condition whereby a height of orthogonality is greater than a predetermined height in an image space.

3. The method according to claim 2, wherein the first-axis positive electrode (111), the second-axis positive electrode (131), and the third-axis positive electrode (121) are the same electrodes.

4. The method according to any one of claims 1 to 3, wherein
one of the electrodes at the intersection of the first observation axis and the body surface of the wearer is located on the back surface of the wearer, and the other one of the electrodes at the intersection of the first observation axis and the body surface of the wearer is located on the front surface of the wearer.

5. The method according to claim 4, wherein the other one electrode is located at a left edge of a fourth intercostal sternum of the wearer.

6. An attachment tool for acquiring cardiac signals, comprising:
- a first-axis positive electrode (111), a first-axis negative electrode (112), a second-axis positive electrode (131), a second-axis negative electrode (132), a third-axis positive electrode (121) and a third-axis negative electrode (122) located on a body surface of a wearer;
- an attachment tool body (140) which fixes the first-axis positive electrode, the first-axis negative electrode, the second-axis positive electrode (131), the second-axis negative electrode (132), the third-axis positive electrode (121) and the third-axis negative electrode (122) to the wearer,
wherein a first-observation axis, which is a straight line connecting the first-axis positive electrode and the first-axis negative electrode, passes through a cardiac apex part or a ventricle anterior wall of the wearer, the second-axis positive electrode (131) being an electrode located at one of the intersections of a second observation axis passing through the cardiac apex part or the ventricle anterior wall of the wearer (9) and the body surface of the wearer, said second observation axis being a non-parallel straight line to the first observation axis; the second-axis negative electrode (132) being an electrode located at the other one of the intersections of the second observation axis and the body surface of the wearer, a third observation axis which is a straight line connecting the third-axis positive electrode and the third-axis negative electrode is a non-parallel straight line to both the first observation axis and the second observation axis, and is substantially parallel to the electromotive force vector of the heart, and -a measuring device (150), wherein the pair of first-axis positive electrode (111) and the first-axis negative electrode (112), the pair of the second-axis positive electrode (131) and the second-axis negative electrode (132), and the pair of the third-axis positive electrode (121) and the third-axis negative electrode (122) are connected via the measuring device (150), , and
- a control unit configured to acquire waveform information of signals propagating along the first, second, and third observation axes, and to output the acquired information to a predetermined output destination.

## Patentansprüche

1. Verfahren zum Erfassen von Herzsignalen, umfassend:
- Anordnen einer ersten Achsen-Pluselektrode (111) und einer ersten Achsen-Negativelektrode (112) an der Oberfläche des Körpers eines Trägers (9), so dass eine erste Beobachtungsachse, bei der es sich um eine gerade Linie handelt, die die erste Achsen-Pluselektrode und die erste Achsen-Negativelektrode verbindet, durch einen Herzapex oder eine Vorderwand des Ventrikels des Trägers (9) verläuft,
- Anordnen einer zweiten Achsen-Pluselektrode (131) und einer zweiten Achsen-Negativelektrode (132) an der Körperoberfläche des Trägers, wobei die zweite Achsen-Pluselektrode (131) eine Elektrode ist, die sich an einem der Schnittpunkte einer zweiten Beobachtungsachse befindet, die durch den Herzapexteil oder die ventrikuläre Vorderwand des Trägers (9) und die Körperoberfläche des Trägers verläuft, wobei die zweite Beobachtungsachse eine nicht parallele gerade Linie zu der ersten Beobachtungsachse ist; und die zweite Achsen-Negativelektrode (132) eine Elektrode ist, die sich an der anderen der Schnittpunkte der zweiten Beobachtungsachse und der Körperoberfläche des Trägers befindet,
- Anordnen einer dritten Achsen-Pluselektrode (121) und einer dritten Achsen-Negativelektrode (122) an der Körperoberfläche des Trägers, so dass eine dritte Beobachtungsachse, die eine gerade Linie ist, die die dritte Achsen-Pluselektrode und die dritte Achsen-Negativelektrode verbindet, eine nicht parallele gerade Linie sowohl zu der ersten Beobachtungsachse als auch zu der zweiten Beobachtungsachse ist und im Wesentlichen parallel zu dem elektromotorischen Kraftvektor des Herzens ist,
wobei das Paar der ersten Achsen-Pluselektrode (111) und der ersten Achsen-Negativelektrode (112), das Paar der zweiten Achsen-Pluselektrode (131) und der zweiten Achsen-Negativelektrode (132) und das Paar der dritten Achsen-Pluselektrode (121) und der dritten Achsen-Negativelektrode (122) über eine Messvorrichtung (150) verbunden sind, und
- wobei eine Steuereinheit so eingerichtet ist, dass sie Wellenforminformationen von Signalen, die sich entlang der ersten, zweiten und dritten Beobachtungsachse ausbreiten, erfasst, und so eingerichtet ist, dass sie die erfassten Informationen an ein vorbestimmtes Ausgabeziel ausgibt.

2. Verfahren nach Anspruch 1, wobei die Anordnung der ersten Beobachtungsachse, der zweiten Beobachtungsachse und der dritten Beobachtungsachse eine Bedingung erfüllt, bei der eine Höhe der Orthogonalität größer als eine vorbestimmte Höhe in einem Bildraum ist.

3. Verfahren nach Anspruch 2, wobei die erste Achsen-Pluselektrode (111), die zweite Achsen-Pluselektrode (131) und die dritte Achsen-Pluselektrode (121) dieselben Elektroden sind.

4. Verfahren nach einem der Ansprüche 1 bis 3, wobei eine der Elektroden an dem Schnittpunkt der ersten Beobachtungsachse und der Körperoberfläche des Trägers auf der Rückseite des Trägers angeordnet ist, und die andere der Elektroden an dem Schnittpunkt der ersten Beobachtungsachse und der Körperoberfläche des Trägers auf der Vorderseite des Trägers angeordnet ist.

5. Verfahren nach Anspruch 4, wobei sich die andere eine Elektrode an einer linken Kante eines vierten interkostalen Sternums des Trägers befindet.

6. Befestigungswerkzeug zum Erfassen von Herzsignalen, umfassend:
- eine erste Achsen-Pluselektrode (111), eine erste Achsen-Negativelektrode (112), eine zweite Achsen-Pluselektrode (131), eine zweite Achsen-Negativelektrode (132), eine dritte Achsen-Pluselektrode (121) und eine dritte Achsen-Negativelektrode (122), die sich auf einer Körperoberfläche eines Trägers befinden;
- einen Befestigungswerkzeugkörper (140), der die erste Achsen-Pluselektrode, die erste Achsen-Negativelektrode, die zweite Achsen-Pluselektrode (131), die zweite Achsen-Negativelektrode (132), die dritte Achsen-Pluselektrode (121) und die dritte Achsen-Negativelektrode (122) an dem Träger fixiert,
wobei eine erste Beobachtungsachse, bei der es sich um eine gerade Linie handelt, die die erste Achsen-Pluselektrode und die erste Achsen-Negativelektrode verbindet, durch einen Herzapexteil oder eine ventrikuläre Vorderwand des Trägers verläuft, wobei die zweite Achsen-Pluselektrode (131) eine Elektrode ist, die sich an einem der Schnittpunkte einer zweiten Beobachtungsachse befindet, die durch den Herzapexteil oder die ventrikuläre Vorderwand des Trägers (9) und die Körperoberfläche des Trägers verläuft, wobei die zweite Beobachtungsachse eine nicht parallele gerade Linie zu der ersten Beobachtungsachse ist; wobei die zweite Achsen-Negativelektrode (132) eine Elektrode ist, die sich an der anderen der Schnittpunkte der zweiten Beobachtungsachse und der Körperoberfläche des Trägers befindet, wobei eine dritte Beobachtungsachse, die eine gerade Linie ist, die die dritte Achsen-Pluselektrode und die dritte Achsen-Negativelektrode verbindet, eine nicht parallele gerade Linie zu sowohl der ersten Beobachtungsachse als auch der zweiten Beobachtungsachse ist und im Wesentlichen parallel zu dem elektromotorischen Kraftvektor des Herzens ist, und
- eine Messvorrichtung (150), wobei das Paar der ersten Achsen-Pluselektrode (111) und der ersten Achsen-Negativelektrode (112), das Paar der zweiten Achsen-Pluselektrode (131) und der zweiten Achsen-Negativelektrode (132) und das Paar der dritten Achsen-Pluselektrode (121) und der dritten Achsen-Negativelektrode (122) über die Messvorrichtung (150) verbunden sind, und
- eine Steuereinheit, die so eingerichtet ist, dass sie Wellenforminformationen von Signalen, die sich entlang der ersten, zweiten und dritten Beobachtungsachse ausbreiten, erfasst und die erfassten Informationen an ein vorbestimmtes Ausgabeziel ausgibt.

## Revendications

1. Procédé pour acquérir des signaux cardiaques, comportant :
- l'agencement d'une électrode positive (111) de premier axe et d'une électrode négative (112) de premier axe sur la surface d'un corps de porteur (9), de sorte qu'un premier axe d'observation, qui est une ligne droite reliant l'électrode positive de premier axe et l'électrode négative de premier axe, traverse un apex cardiaque ou une paroi antérieure du ventricule du porteur (9),
- l'agencement d'une électrode positive (131) de deuxième axe et d'une électrode négative (132) de deuxième axe sur la surface corporelle du porteur, ladite électrode positive (131) de deuxième axe étant une électrode située à l'une des intersections d'un deuxième axe d'observation traversant la partie apex cardiaque ou la paroi antérieure ventriculaire du porteur (9) et la surface corporelle du porteur, ledit deuxième axe d'observation étant une ligne droite non parallèle au premier axe d'observation ; et ladite électrode négative (132) de deuxième axe étant une électrode située à l'autre des intersections du deuxième axe d'observation et de la surface corporelle du porteur,
- l'agencement d'une électrode positive (121) de troisième axe et d'une électrode négative (122) de troisième axe sur la surface corporelle du porteur, de sorte qu'un troisième axe d'observation qui est une ligne droite reliant l'électrode positive de troisième axe et l'électrode négative de troisième axe est une ligne droite non parallèle à la fois au premier axe d'observation et au deuxième axe d'observation, et est sensiblement parallèle au vecteur de force électromotrice du cœur,
dans lequel la paire de la première électrode positive (111) et de la première électrode négative (112), la paire de la deuxième électrode positive (131) et de la deuxième électrode négative (132), et la paire de la troisième électrode positive (121) et de la troisième électrode négative (122) sont connectées par le biais d'un dispositif de mesure (150), et
- dans lequel une unité de commande est configurée pour acquérir des informations de forme d'onde de signaux se propageant le long des premier, deuxième et troisième axes d'observation, et est configurée pour sortir les informations acquises vers une destination de sortie prédéterminée.

2. Procédé selon la revendication 1, dans lequel l'agencement du premier axe d'observation, du deuxième axe d'observation et du troisième axe d'observation satisfait à une condition selon laquelle une hauteur d'orthogonalité est supérieure à une hauteur prédéterminée dans un espace d'image.

3. Procédé selon la revendication 2, dans lequel l'électrode positive (111) de premier axe, l'électrode positive (131) de deuxième axe et l'électrode positive (121) de troisième axe sont les mêmes électrodes.

4. Procédé selon l'une quelconque des revendications 1 à 3, dans lequel
l'une des électrodes à l'intersection du premier axe d'observation et de la surface corporelle du porteur est située sur la surface arrière du porteur, et l'autre des électrodes à l'intersection du premier axe d'observation et de la surface corporelle du porteur est située sur la surface avant du porteur.

5. Procédé selon la revendication 4, dans lequel l'autre électrode est située au niveau d'un bord gauche d'un quatrième sternum intercostal du porteur.

6. Outil de fixation pour acquérir des signaux cardiaques, comportant :
- une électrode positive (111) de premier axe, une électrode négative (112) de premier axe, une électrode positive (131) de deuxième axe, une électrode négative (132) de deuxième axe, une électrode positive (121) de troisième axe et une électrode négative (122) de troisième axe situées sur une surface corporelle d'un porteur ;
- un corps d'outil de fixation (140) qui fixe l'électrode positive de premier axe, l'électrode négative de premier axe, l'électrode positive (131) de deuxième axe, l'électrode négative (132) de deuxième axe, l'électrode positive (121) de troisième axe et l'électrode négative (122) de troisième axe à l'utilisateur,
dans lequel un premier axe d'observation, qui est une ligne droite reliant l'électrode positive de premier axe et l'électrode négative de premier axe, traverse une partie apex cardiaque ou une paroi antérieure ventriculaire du porteur, l'électrode positive (131) de deuxième axe étant une électrode située à l'une des intersections d'un deuxième axe d'observation traversant la partie apex cardiaque ou la paroi antérieure ventriculaire du porteur (9) et la surface corporelle du porteur, ledit deuxième axe d'observation étant une ligne droite non parallèle au premier axe d'observation ; l'électrode négative (132) de deuxième axe étant une électrode située à l'autre des intersections du deuxième axe d'observation et de la surface corporelle du porteur, un troisième axe d'observation qui est une ligne droite reliant l'électrode positive de troisième axe et l'électrode négative de troisième axe est une ligne droite non parallèle à la fois au premier axe d'observation et au deuxième axe d'observation, et est sensiblement parallèle au vecteur de force électromotrice du cœur, et - un dispositif de mesure (150), dans lequel la paire d'électrode positive (111) de premier axe et de l'électrode négative (112) de premier axe, la paire de l'électrode positive (131) de deuxième axe et de l'électrode négative (132) de deuxième axe, et la paire de l'électrode positive (121) de troisième axe et de l'électrode négative (122) de troisième axe sont connectées par le biais du dispositif de mesure (150), et
- une unité de commande configurée pour acquérir des informations de forme d'onde de signaux se propageant le long des premier, deuxième et troisième axes d'observation, et pour sortir les informations acquises vers une destination de sortie prédéterminée.
